(19) Europäisches Patentamt
European Patent Office
Office européen des brevets

(11) **EP 4 512 438 A1**

(12) **EUROPEAN PATENT APPLICATION**
published in accordance with Art. 153(4) EPC

(43) Date of publication:
**26.02.2025 Bulletin 2025/09**

(21) Application number: **23792016.0**

(22) Date of filing: **03.03.2023**

(51) International Patent Classification (IPC):
**A61L 27/36** (2006.01)    **A61L 27/40** (2006.01)

(52) Cooperative Patent Classification (CPC):
**A61L 27/36; A61L 27/40**

(86) International application number:
**PCT/KR2023/002941**

(87) International publication number:
**WO 2023/204435 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.04.2022 KR 20220049750**

(71) Applicant: **T&R Biofab Co., Ltd.**
**Siheung-si Gyeonggi-do 15111 (KR)**

(72) Inventors:
• **KIM, Hyun Jung**
**Uiwang-si, Gyeonggi-do 16024 (KR)**

• **KIM, Chang Hwan**
**Siheung-si, Gyeonggi-do 14985 (KR)**
• **PARK, Eun Hye**
**Ansan-si, Gyeonggi-do 15521 (KR)**
• **KIM, Young Hwan**
**Suwon-si, Gyeonggi-do 16313 (KR)**
• **SHIN, Jae Hang**
**Siheung-si, Gyeonggi-do 15032 (KR)**
• **CHOI, In Hank**
**Hwaseong-si, Gyeonggi-do 18585 (KR)**

(74) Representative: **Caspary, Karsten et al**
**Kroher-Strobel**
**Rechts- und Patentanwälte PartmbB**
**Bavariaring 20**
**80336 München (DE)**

(54) **TISSUE MATRIX DECELLULARIZED IN MULTIPLE STAGES, AND PREPARATION METHOD THEREFOR**

(57)    The present invention relates to a multi-stage decellularized mammal-derived tissue matrix, prepared by a method comprising: a preparation step for preparing tissue of a non-human mammal; a pretreatment step for pretreating the tissue; a first inactivation step for inactivating a virus included in the pretreated tissue using alcohol; a primary decellularization step for removing cells from the virus-inactivated tissue using an aqueous basic solution; a secondary decellularization step for removing cells by enzymatically treating the primary decellularized tissue; and a second inactivation step for inactivating a virus included in the decellularized tissue using an acid, and wherein the DNA content in the tissue subjected to the second inactivation step is 50 ng/mg or less, and the reduction rate (L) of the elastin content in the tissue subjected to the pretreatment step to the second inactivation step is 20% or less; and a method for preparing the same. The tissue matrix of the present invention has the advantage that immune responses or foreign body reactions are minimized since the DNA content is very low, and the wound healing and scar suppression effects are high since the elastin content is high.

[Figure 1]

EP 4 512 438 A1

**Description**

[Technical Field]

The present invention relates to a multi-stage decellularized tissue matrix and a method for preparing the same, and more specifically, to a multi-stage decellularized tissue matrix having a high elastin content and capable of improving productivity due to high decellularization efficiency, and a method for preparing the same.

[Background Art]

The extracellular matrix (ECM) is a three-dimensional network structure that exists outside the cell and contains components such as collagen, elastin, fibronectin, laminin and GAG, closely associated with the cell, and plays a role in protecting and supporting cells by filling the space between cells and tissues, is used as a signaling pathway when cells interact with components in the extracellular matrix through cell receptors such as integrins, and has various other functions.

Specifically, the extracellular matrix regulates the function and role of cells by capturing growth factors, enzymes, and other molecules involved in cell activity, and collagen and fibronectin contained in the extracellular matrix provide the physical strength necessary for cells to maintain their shape and move.

Meanwhile, elastin and laminin provide elasticity and play a role in regulating tissues and organs to perform specific functions since specific components are distributed within the extracellular matrix depending on the human tissue and organ.

In particular, elastin contained in the extracellular matrix not only provides elasticity to the extracellular matrix, but also acts as a cause of scar formation because when the rate of elastin production is low during the healing process of damaged tissue, it fails to be restored to the same structure as the original tissue, and therefore, it is considered a very important substance in wound healing and tissue regeneration and is widely applied in the fields of medical devices and regenerative medicine.

In general, when artificial tissues or organs for transplantation or research, or organ models for new drug development are prepared, biocompatible materials that are nontoxic and do not induce immune responses are used. Previously, biocompatible polymers such as poly latic acid (PLA) or poly glycolic acid (PGA) were used, but recently, animal tissue-derived biomaterials have been in the spotlight, and biomaterials based on decellularized extracellular matrix (dECM) from such animal tissue-derived biomaterials are mainly used.

As such, biomaterials derived from animal tissues contain substances that induce immune or inflammatory responses, such as cells, fats, viruses, and other foreign substances, and therefore, the most important part in preparing biomaterials derived from animal tissues is the process of removing these foreign substances. To remove these substances, acid, alkali or enzyme treatments are generally used, but no method has yet been developed that can efficiently remove all cells, crude fats, viruses and other foreign substances, and conventional methods has disadvantages in that the yield of biomaterials is low, processing time is long, and elastin content is low.

[Prior Art References]

[Patent Documents]

(Patent Document 1) Korean Patent No. 10-1410533 (Registered on June 16, 2014)

[Disclosure]

[Technical Problem]

The present invention aims to provide a multi-stage decellularized tissue matrix having a high elastin content and capable of improving productivity due to high decellularization efficiency, and a method for preparing the same.

[Technical Solution]

One embodiment of the present invention for achieving the above-described purposes relates to a multi-stage decellularized mammal-derived tissue matrix, prepared by a method comprising: a preparation step for preparing tissue of a non-human mammal; a pretreatment step for pretreating the tissue; a first inactivation step for inactivating a virus included in the pretreated tissue using alcohol; a primary decellularization step for removing cells from the virus-inactivated tissue using an aqueous basic solution; a secondary decellularization step for removing cells by enzymatically treating the primary decellularized tissue; and a second inactivation step for inactivating a virus included in the decellularized tissue using an acid, and wherein the DNA content in the tissue subjected to the second inactivation step is 50 ng/mg or less, wherein the reduction rate (L) of the elastin content in the tissue subjected to the pretreatment step to the second inactivation step is 20% or less.

The primary decellularization step may comprise a first decellularization step using a mixture of a aqueous basic solution and an alcohol; and a second decellularization step using a aqueous basic solution.

The tissue may be any one or more of mammal-derived blood vessel, ligament and tendon.

The first decellularization step may be performed using a mixture of n-PrOH and NaOH, and the second decellularization step may be performed using an aqueous sodium hydroxide solution of more than 0.05 M and less than 0.2 M.

The secondary decellularization step may be performed using DNase.

Another embodiment of the present invention relates to a method for preparing a decellularized tissue matrix, and specifically, comprises: a preparation step for preparing tissue of a non-human mammal; a pretreatment step for pretreating the tissue; a first inactivation step for inactivating a virus included in the pretreated tissue using alcohol; a primary decellularization step for removing cells from the virus-inactivated tissue using an aqueous basic solution; a secondary decellularization step for removing cells by enzymatically treating the primary decellularized tissue; and a second inactivation step for inactivating a virus included in the decellularized tissue using an acid.

In this case, the DNA content in the tissue subjected to the second inactivation step may be 50 ng/mg or less, and the reduction rate (L) of the elastin content in the tissue subjected to the pretreatment step to the second inactivation step may be 20% or less.

A neutralization step using an acidic solution and a washing step using PBS may be further performed between the primary and secondary decellularization steps.

A decolorization step and/or a defatization step may be further performed between the secondary decellularization step and the second inactivation step.

The second inactivation step may be performed using a mixture of an organic acid and an alcohol.

Washing, packaging and sterilization steps may be performed after the second inactivation step.

[Advantageous Effects]

The multi-stage decellularized tissue matrix of the present invention has a low rate of elastin loss during the decellularization process, and thus may promote re-epithelialization and granulation tissue and new blood vessel formation of damaged tissue due to elastin, thereby helping to rapidly regenerate tissue.

The method for preparing a multi-stage decellularized tissue matrix of the present invention has excellent decellularization efficiency and a short process time, and thus may remarkably improve the productivity of the multi-stage decellularized tissue matrix.

[Description of Drawings]

Figures 1(A) and 1(B) are graphs showing the experimental results of Experimental Example 1.
Figure 2 is a graph showing the experimental results of Experimental Example 2.
Figures 3(A) and 3(B) are graphs showing the experimental results of Experimental Example 3.
Figures 4(A) and 4(B) are graphs showing the experimental results of Experimental Example 4.
Figure 5 is a table showing the experimental results of Experimental Example 5.

[Best Mode]

Before explaining in detail the preferred embodiments of the present invention below, it should be noted that the terms and words used in this specification and claims should not be interpreted as limited to their usual or dictionary meanings, but should be interpreted as meanings and concepts consistent with the technical spirit of the present invention.

Throughout this specification, when a part is said to "comprise" or "include" an element, it means that other elements may be further included without excluding other elements, unless otherwise stated.

Throughout this specification, "%" used to indicate the concentration of a particular substance means (weight/weight) % for solid/solid, (weight/volume) % for solid/liquid, and (volume/volume)% for liquid/liquid, unless otherwise stated.

In addition, throughout this specification, the term "mammal" refers to a non-human mammal, and should be understood to mean a non-human mammal even if the "mammal" is simply described without mentioning that a human is excluded, and the expression "cells are removed" should be understood to have an inclusive meaning including that DNA is destroyed or removed.

In addition, each of the steps may be performed in a different order than specified, unless a specific order is clearly described from the context. That is, each of the steps may be performed in the same order as specified, may be performed substantially simultaneously, or may be performed in the opposite order.

In addition, unless otherwise defined, all technical and scientific terms used in this specification have the same meaning as commonly understood by those of ordinary skill in the technical field to which the present invention pertains, and in case of conflict, the description in this specification, including definitions, shall prevail.

Hereinafter, embodiments of the present invention will be described. However, the scope of the present invention is not limited to the preferred embodiments below, and those skilled in the art can implement various modified forms of the contents described in this specification within the scope of the present invention.

The present invention relates to a multi-stage decellularized tissue matrix and a method for preparing the same, and specifically, to a multi-stage decellularized tissue matrix, in which cells, crude fats, viruses and other foreign substances in mammal-derived tissues are effectively removed through a multi-stage decellularization process, and the effects of promoting tissue regeneration and suppressing scar formation due to elastin are maximized because elastin loss is reduced during the decellularization process, and a method for preparing the same.

First, One embodiment of the present invention relates to a multi-stage decellularized tissue matrix, and the multi-stage decellularized mammal-derived tissue matrix according to one embodiment of the present invention is prepared by a method comprising: a preparation step for preparing tissue of a non-human mammal; a pretreatment step for pretreating the tissue; a first inactivation step for inactivating a virus included in the pretreated tissue using alcohol; a primary decellularization step for removing cells from the virus-inactivated tissue using an aqueous basic solution; a secondary decellularization step for removing cells by enzymatically treating the primary decellularized tissue; and a second inactivation step for inactivating a virus included in the decellularized tissue using an acid.

In this case, the DNA content in the tissue subjected to the second inactivation step may be 50 ng/mg or less, preferably 20 ng/mg or less, 10 ng/mg or less, and more preferably 5 ng/mg or less. In addition, the reduction rate (L) of the elastin content in the tissue subjected to the pretreatment step to the second inactivation step is 20% or less.

In this case, the reduction rate (L) is defined by the following equation (1), wherein $L_0$ represents the elastin content in the tissue in the preparation step, and $L_f$ represents the elastin content in the tissue subjected to the second inactivation step.

$$L(\%) = \frac{(L_0 - L_f)}{L_0} \times 100 \qquad \{\text{Equation (1)}\}$$

First, the preparation step is a step of preparing tissue of a non-human mammal. In this case, the mammal may be a non-human mammal, for example, a pig, a horse, a cow, a sheep, etc., and the tissue may be any one or more of blood vessel, ligament and tendon derived from such mammals.

To prepare a multi-stage decellularized tissue matrix, mammal tissue is first collected. The mammal tissue thus collected is treated by removing useless tissue and blood attached to the mammal tissue, washing, drying and cutting in the preparation step. The mammal tissue thus prepared may be stored frozen and may be thawed and used when work is required. When frozen raw material tissue is used, the preparation step may be a step of taking out and preparing the frozen raw material tissue.

Next, the mammal tissue prepared in the preparation step is pretreated through the pretreatment step.

This step is a step of washing the mammal tissue, and if the mammal tissue is frozen, it may be thawed, washed and cut through this step. In this step, distilled water, purified water, saline solution, etc. may be used as the washing water, and preferably, distilled water is used. Physical stirring may be performed from this step to the second inactivation step.

The mammal tissue prepared through the pretreatment step may be subjected to the first inactivation step, primary decellularization step, secondary decellularization step and second inactivation step, thereby removing cells, crude fats, viruses and other foreign substances that may induce immune responses and foreign body reactions. By decellularization through such a multi-stage reaction, the decellularization efficiency and foreign substance removal efficiency at each step are remarkably improved, so that the amount of tissue processed at one time increases and the processing time is shortened, and as a result, the productivity and yield of the decellularized tissue matrix may be improved.

First, the first inactivation step is a step of inactivating a virus included in the pretreated tissue using alcohol.

This step is performed to more effectively remove cells, fats and foreign substances in the primary and secondary decellularization steps by primarily inactivating a virus within the tissue prior to decellularization of the tissue.

The alcohol used in this step may be n-propanol, and an aqueous n-propanol solution with a concentration of 50 to 90% is preferably used to obtain a virus inactivation effect, and an aqueous n-propanol solution with a concentration of 65 to 80% may be more preferably used.

In the present invention, n-propanol is used as the alcohol for virus inactivation in the first inactivation step. If n-propanol is used for virus inactivation in this way, the tissue becomes softer, and when the tissue is soft, the treatment solution penetrates better into the tissue, and thus, an effect is obtained that allows for overall uniform performance of virus inactivation and decellularization. As a result, the efficiency of inactivation and decellularization treatment is improved, more uniform tissue may be obtained, and the quality and reliability of the finally obtained decellularized tissue matrix may be improved.

Ethanol, which is generally used for virus inactivation, has the characteristic of fixing and hardening the mammal tissue to be decellularized, and when ethanol is used instead of n-propanol in this step of the present invention, it is not preferable because the tissue becomes hard, and thus, the efficiency of the process of loosening the tissue to improve the penetration ability of the treatment material in the subsequent step decreases.

In addition, iso-propanol has a low virus inactivation effect, and if iso-propanol is used in the first inactivation step, since sufficient virus inactivation effect is not obtained, the decellularization efficiency is reduced in the subsequent primary and secondary decellularization steps, and therefore, it is preferable to use n-propanol in the first inactivation step.

In this step, the pretreated tissue may be treated in an amount of 10 to 25 parts by weight, preferably 20 to 25 parts by weight, based on 100 parts by weight of the aqueous propanol solution.

The primary decellularization step is a step of treating the virus-inactivated tissue from the first inactivation step with an aqueous basic solution to remove cells.

This step comprises a first decellularization step for first treating the tissue using a mixture of an aqueous basic solution and alcohol; and a second decellularization step for treating the tissue using an aqueous basic solution. In this case, the aqueous basic solution is preferably an aqueous sodium hydroxide solution, and the alcohol is preferably n-propanol, and in this case, the first decellularization step is the step performed by treating the tissue with a mixture of n-propanol and sodium hydroxide, and the second decellularization step is the step performed by treating the tissue with an aqueous sodium hydroxide solution.

When the decellularization step is performed in two steps like this, since not only is decellularization performed, but also foreign substances such as crude fat are removed through the first decellularization step, a more effective decellularization process is performed in the second decellularization step.

In each of the first and second decellularization steps, the tissue may be treated in an amount of 10 to 25 parts by weight, preferably 20 to 25 parts by weight, based on 100 parts by weight of the treatment solution, which is nearly twice the conventional treatment capacity, and since effective decellularization may be performed through the multi-stage method of the present invention, there is an effect that allows for sufficient treatment of twice the amount of tissue compared to the conventional method.

Specifically, the first decellularization step is a step of treating the virus-inactivated tissue with a mixture of n-propanol and sodium hydroxide. In this step, the crude fat and sodium hydroxide in the tissue undergo a saponification reaction, and the crude fat is separated and removed from the tissue, wherein n-propanol promotes this saponification reaction and acts to cause the products of the saponification reaction to coagulate with each other, allowing the crude fat to be separated and removed from the tissue more quickly and efficiently.

In this case, the treatment time for treating the tissue with a mixture of n-propanol and sodium hydroxide may be 10 to 48 hours, preferably 15 to 40 hours, and more preferably 18 to 28 hours, and the treatment temperature may be 0 to 30°C, preferably 10 to 28°C, and more preferably 18 to 25°C. When treated within this temperature range for this time, sufficient crude fat removal efficiency may be obtained without significant damage to the tissue structure or loss of elastin.

The mixture of n-propanol and sodium hydroxide may be an aqueous solution with a concentration of n-propanol of 50 to 95%, preferably 60 to 90%, more preferably 65 to 85%, and a concentration of sodium hydroxide of more than 0.05 M and less than 0.2 M. These concentration ranges are concentration ranges that may minimize tissue damage while at the same time effectively removing foreign substances such as crude fat in the first decellularization step, and in particular, if the concentration of sodium hydroxide is less than this range, the treatment efficiency of the primary decellularization step is reduced, and if it is outside this range, there is a problem that the tissue structure itself collapses and dissolves, and therefore, it is preferable to use an n-propanol and aqueous sodium hydroxide solution in the concentration ranges described above.

The second decellularization step is a step for simultaneously causing a decellularization reaction while loosening the structure of the tissue by treating the tissue subjected to the first decellularization step with an aqueous basic solution to soften the tissue. Crude fats and various foreign substances included in the tissue act as a kind of physical and chemical barrier in the decellularization reaction, and since the tissue was subjected to the first decellularization step and the crude fats and various foreign substances were removed, the decellularization reaction may be performed more effectively in the second decellularization step.

In this step, the treatment time for treating the tissue with an aqueous basic solution may be 10 to 48 hours, preferably 15 to 40 hours, and more preferably 18 to 28 hours, and the treatment temperature may be 0 to 30°C, preferably 10 to 28°C, and more preferably 18 to 25°C, and when treated within this temperature range for this time, the tissue structure may be sufficiently loosely deformed without collapse of the tissue structure or loss of elastin, and an appropriate decellularization reaction may occur.

In this case, the aqueous basic solution used as the treatment solution may be an aqueous sodium hydroxide solution, and the concentration of sodium hydroxide included in the aqueous sodium hydroxide solution may be more than 0.05 M and less than 0.2 M. If the concentration of sodium hydroxide is 0.05 M or less, since the tissue structure is not sufficiently loosened, there is a problem that the decellularization efficiency decreases in the secondary decellularization step described later, and if it is 0.2 M or more, since some tissues may collapse or dissolve beyond the level of loosening in this step, the final yield may decrease remarkably and elastin may be lost, and therefore, it is preferable to use an aqueous sodium hydroxide solution of the concentration described above.

The secondary decellularization step using enzymes is performed after the primary decellularization step, and a neutralization step and a washing step may be performed between these two steps.

The neutralization step is a step of treating the tissue with an aqueous acidic solution to neutralize the sodium hydroxide used in the primary decellularization step, and an aqueous acidic solution containing any one or more of, for example, but not limited to, hydrochloric acid, sulfuric acid, acetic acid and peracetic acid may be used as the type of aqueous acidic solution used, and the concentration thereof may also be appropriately adjusted and used according to the working environment or conditions.

The washing step is the step performed to prevent a decrease in enzyme reactivity due to residues when enzymatically treated in the subsequent secondary decellularization step, and a buffer solution may be used as the washing solution used in the washing step, and for example, a phosphate buffered saline (PBS) solution may be used, but is not limited.

Meanwhile, the secondary decellularization step is a step of enzymatically treating the primary decellularized tissue to remove cells, and the step of treating the primary decellularized tissue with a DNA-degrading enzyme to remove DNA within the primary decellularized tissue.

In this step, the tissue may be treated in an amount of 10 to 25 parts by weight, preferably 20 to 25 parts by weight, based on 100 parts by weight of the treatment solution containing the DNA-degrading enzyme, which is nearly twice the conventional treatment capacity as described above and is an achievable treatment capacity because the treatment efficiency is increased by the multi-stage decellularization treatment process of the present invention.

In this step, the treatment time for treating the tissue with the DNA-degrading enzyme to obtain enzyme activity and sufficient DNA degradation efficiency may be 10 to 35 hours, preferably 18 to 30 hours, and the treatment temperature may be 30 to 45°C, preferably 35 to 42°C.

In the present invention, the concentration of the DNA-degrading enzyme contained in the treatment solution is 0.0001 to 0.005 wt%, which is several to hundreds of times lower than the concentration of DNA-degrading enzyme typically used in conventional similar technologies. The primary decellularized tissue of the present invention is subjected to the primary decellularization step, wherein foreign substances including crude fats within the tissue are removed and the structure of the tissue becomes loose. In this state, when a DNA-degrading enzyme is added, the DNA-degrading enzyme may penetrate very easily into the tissue structure, and accordingly, even if a low concentration of the DNA-degrading enzyme is used, at least the same or better DNA removal efficiency may be secured, and therefore, a low concentration of the DNA-degrading enzyme as described above may be used in the present invention.

However, if the concentration of the DNA-degrading enzyme is less than this rage, the DNA degradation efficiency decreases, and therefore, it is preferable to use it at a concentration of 0.0001% or more, and if it exceeds this range, since the degree of improvement in DNA degradation efficiency is extremely small compared to the amount of the added DNA-degrading enzyme, it is uneconomical, and therefore, it is preferable to use the DNA-degrading enzyme in the concentration range described above.

The decellularized tissue from the secondary decellularization step is subjected to the second inactivation step to remove an additional virus, and additional decolorization and/or defatization steps may be performed between these two steps. The decolorization step is a step of removing the color of the tissue, and may be performed, for example, by treating the tissue with hydrogen peroxide, and the defatization step is a step of further removing fat included in the tissue, and may be performed, for example, using a ketone solution, preferably an acetone solution.

Subsequently, the second inactivation step is performed to further inactivate a virus included within the tissue subjected to the secondary decellularization step. This step is a step of inactivating a virus within the tissue using an acid, specifically the step of treating the tissue with a mixture of an organic acid and an alcohol.

In this case, the organic acid used may be peracetic acid, wherein the peracetic acid is an oxidizing agent and may inactivate a virus such a way as to cause non-specific free radical damage to the virus. The peracetic acid used in this step may be used at a concentration of 0.05 to 1%.

In addition, the alcohol used in this step may be a lower alcohol having 1 to 4 carbon atoms, such as methanol, ethanol and propanol, and preferably, may be ethanol. Ethanol has the function of inactivating a virus and the function of fixing a tissue structure to make the tissue firm, and thus, it is preferable to use ethanol.

Through these steps, the virus is finally inactivated, the crude fat and DNA are removed, and a tissue matrix comprising various extracellular matrix components including elastin may be obtained.

The tissue matrix thus obtained has a very low DNA content of 50 ng/mg or less, preferably 20 ng/mg or less, 10 ng/mg or less, and more preferably 5 ng/mg or less, so that the induction of immune responses or various foreign body reactions may be minimized.

In addition, the tissue matrix has the advantage that it has effects such as wound healing and scar suppression due to elastin because the elastin content that has reduced through the pretreatment step to second inactivation step is very low. Specifically, the reduction rate (L) of the elastin content in the tissue subjected to the pretreatment step to the second inactivation step is 20% or less. This numerical value is very low compared to the very high reduction rate (L) of elastin content of 50% or more when the conventional decellularization method is applied.

Meanwhile, additional steps such as washing, packaging, sterilization and processing may be performed after the second inactivation step. The washing may be performed using a buffer solution, distilled water, etc., and the packaging is performed to prevent contamination and facilitate handling during storage or transport of the tissue matrix, and it may be packaged immersed in a preservative solution when packaged.

The sterilization is performed to remove any additional contamination that may occur after the second inactivation step, and for example, but not limited to, electron beam irradiation or radiation irradiation may be performed for sterilization.

The processing refers to processing the tissue matrix into a usable form, and for example, methods such as drying, powdering and solutionization may be applied, and it may processed in this way and utilized in the form of bioink or injection, and may be utilized in various forms without being limited to these.

Meanwhile, another embodiment of the present invention relates to a method for preparing a mammal-derived tissue matrix, and some of the description overlapping with one embodiment of the present invention is omitted.

Specifically, the method for preparing a decellularized tissue matrix comprises: a preparation step for preparing tissue of a non-human mammal; a pretreatment step for pretreating the tissue; a first inactivation step for inactivating a virus included in the pretreated tissue using alcohol; a primary decellularization step for removing cells from the virus-inactivated tissue using an aqueous basic solution; a secondary decellularization step for removing cells by enzymatically treating the primary decellularized tissue; and a second inactivation step for inactivating a virus included in the decellularized tissue using an acid.

In this case, the DNA content in the tissue subjected to the second inactivation step may be 50 ng/mg or less, and the reduction rate (L) of the elastin content in the tissue subjected to the pretreatment step to the second inactivation step may be 20% or less.

In this case, the reduction rate (L) of the elastin content in the tissue is defined by the following equation (1), wherein $L_0$ is the elastin content in the tissue in the preparation step, and $L_f$ is the elastin content in the tissue subjected to the second inactivation step.

$$L(\%) = \frac{(L_0 - L_f)}{L_0} \times 100 \qquad \{Equation\ (1)\}$$

First, the preparation step is a step of preparing tissue of a non-human mammal. In this case, the mammal may be a non-human mammal, for example, a pig, a horse, a cow, a sheep, etc., and the tissue may be any one or more of blood vessel, ligament and tendon derived from such mammals.

The pretreatment step is a step of washing the mammal tissue prepared in the preparation step, and the tissue may be cut into an appropriate size at this step, if necessary, and when previously frozen tissue is prepared, thawing may be performed at this step.

The first inactivation step is a step of inactivating a virus included in the pretreated tissue using alcohol, and ethanol is generally used for virus inactivation, n-propanol is used in the present invention. This is because ethanol firmly fixes the tissue to reduce the softening efficiency in the subsequent step, whereas n-propanol softens the tissue to improve the softening efficiency in the subsequent step.

The primary decellularization step is a step of treating the virus-inactivated tissue from the first inactivation step with an aqueous basic solution to remove cells. This step comprises a first decellularization step for first treating the tissue using a mixture of an aqueous basic solution and alcohol; and a second decellularization step for treating the tissue using an aqueous basic solution.

In this case, the aqueous basic solution is an aqueous sodium hydroxide solution, and it is preferable use n-propanol as the alcohol.

Specifically, the first decellularization step is a step of treating the virus-inactivated tissue with a mixture of n-propanol and sodium hydroxide, and through this step, crude fats and foreign substances within the tissue are removed and the tissue is partially softened. The second decellularization step is a step of treating the tissue with an aqueous sodium hydroxide solution, and in this step, a decellularization reaction occurs simultaneously with tissue softening, and this effect may be further enhanced by removing foreign substances including crude fat in advance.

In the first and second decellularization steps, sodium hydroxide may be included in a concentration of more than 0.05 M and less than 0.2 M, which is a desirable concentration range for preventing collapse or dissolution of the tissue while sufficiently causing saponification and softening reactions by sodium hydroxide.

Next, the secondary decellularization step using enzymes is performed after the primary decellularization step, and a neutralization step and a washing step may be performed between these two steps. The neutralization step is a step of treating the tissue with an aqueous acidic solution to neutralize the sodium hydroxide used in the primary decellularization step, and the washing step is a step performed to prevent a decrease in enzyme reactivity due to residues when enzymatically treated in the subsequent secondary decellularization step.

The secondary decellularization step is a step of treating the primary decellularized tissue with a DNA-degrading enzyme to remove DNA within the tissue. The primary decellularized tissue of the present invention is subjected to the primary decellularization step, wherein foreign substances including crude fats within the tissue are removed and the structure of the tissue becomes loose. In this state, when a DNA-degrading enzyme is added, the DNA-degrading enzyme may penetrate very easily into the tissue structure, and accordingly, even if a low concentration of the DNA-degrading enzyme is used, at least the same or better DNA removal efficiency may be secured, and therefore, the DNA-degrading enzyme is used at a low concentration of 0.0001 to 0.005 w% in the present invention. If the concentration of the DNA-degrading enzyme is less than this rage, the DNA degradation efficiency decreases, and if it exceeds this range, since the degree of improvement in DNA degradation efficiency is extremely small compared to the amount of the added DNA-degrading enzyme, it is uneconomical, and therefore, it is preferable to use the DNA-degrading enzyme in the concentration range described above.

The decellularized tissue from the secondary decellularization step is subjected to the second inactivation step to remove an additional virus, and additional decolorization and/or defatization steps may be performed between these two steps. The decolorization step is a step of removing the color of the tissue, and may be performed, for example, by treating the tissue with hydrogen peroxide, and the defatization step is a step of further removing fat included in the tissue, and may be performed, for example, using a ketone solution, preferably an acetone solution.

Subsequently, the second inactivation step is performed to further inactivate a virus included within the tissue subjected to the secondary decellularization step. This step is a step of treating the tissue with a mixture of an organic acid and an alcohol, wherein a peracetic acid may be used as the organic acid and ethanol may be used as the alcohol, and a virus within the tissue may be effectively removed by treating the tissue with this mixed solution.

Through these steps, the virus is finally inactivated, the crude fat and DNA are removed, and a tissue matrix comprising various extracellular matrix components including elastin may be prepared.

The tissue matrix thus obtained has a very low DNA content of 50 ng/mg or less, preferably 20 ng/mg or less, 10 ng/mg or less, and more preferably 5 ng/mg or less, so that the induction of immune responses or various foreign body reactions may be minimized.

In addition, the tissue matrix has the advantage that it has effects such as wound healing and scar suppression due to elastin because the elastin content that has reduced through the pretreatment step to second inactivation step is very low. Specifically, the reduction rate (L) of the elastin content in the tissue subjected to the pretreatment step to the second inactivation step is 20% or less. This numerical value is very low compared to the very high reduction rate (L) of elastin content of 50% or more when the conventional decellularization method is applied.

Meanwhile, additional steps such as washing, packaging, sterilization and processing may be performed after the second inactivation step. The washing may be performed using a buffer solution, distilled water, etc., the packaging is performed to prevent contamination and facilitate handling during storage or transport of the tissue matrix, and the sterilization is performed to remove any additional contamination that may occur after the second inactivation step.

The processing refers to processing the tissue matrix into a usable form, and for example, methods such as drying, powdering and solutionization may be applied, and it may processed in this way and utilized in the form of bioink or injection, and may be utilized in various forms without being limited to these.

Hereinafter, the specific action and effect of the present invention will be explained through an example of the present invention. However, this is presented as a preferred example of the present invention, and the scope of the present invention is not limited according to the example.

**[Preparative Example]**

1. Preparation and pretreatment steps

First, the washed, dried, cut and frozen porcine aorta attached to the heart was prepared, thawed, washed with distilled water, and cut to a size of 50×50 mm to prepare raw material tissue specimens.

2. First inactivation step

Next, 250 g of the raw material tissue specimen was added to 1 L of 70% n-propanol and stirred at a temperature of 20°C and a stirring speed of 150 RPM for 24 hours to perform the first inactivation step.

3-1. Primary decellularization step (first decellularization step)

Next, a mixed solution containing 70% concentration of n-propanol and 0.1 M concentration of sodium hydroxide in distilled water was prepared, and the raw material tissue specimen was mixed to contain 23 parts by weight based on 100 parts by weight of the mixed solution, and then stirred at a temperature of 20°C and a stirring speed of 150 RPM for 24 hours to perform the first decellularization step.

3-2. Primary decellularization step (second decellularization step)

Next, the tissue specimen subjected to the first decellularization step was mixed to contain 24 parts by weight based on 100 parts by weight of a pre-prepared 0.1 M aqueous sodium hydroxide solution, and then stirred for 24 hours under the same conditions as the previous step to perform the second decellularization step.
Subsequently, the tissue specimen subjected to the second decellularization step was immersed in an aqueous acetic acid solution, stirred to neutralize, and then washed with a PBS solution.

4. Secondary decellularization step

Subsequently, the tissue specimen was placed in a treatment solution containing DNase at a concentration of 0.0001 wt% and stirred at a temperature of 37°C and a stirring speed of 150 RPM for 23 hours to perform a secondary decellularization step. The tissue specimen subjected to the secondary decellularization step was decolorized by treating it in 3% hydrogen peroxide solution at 20°C for 1 hour, and defatized by treating it in 20% aqueous acetone solution at 20°C for 1 hour.

5. Second inactivation step

Subsequently, the tissue specimen was immersed in a mixed solution containing 70% concentration of n-propanol and 0.2% peracetic acid in distilled water, and treated at 20°C for 4 hours and a half to perform a second inactivation step.
Finally, the tissue specimen subjected to all of the processing steps was washed with PBS solution and distilled water to prepare the multi-stage decellularized tissue matrix according to one embodiment of the present invention.

**[Experimental Example 1]**

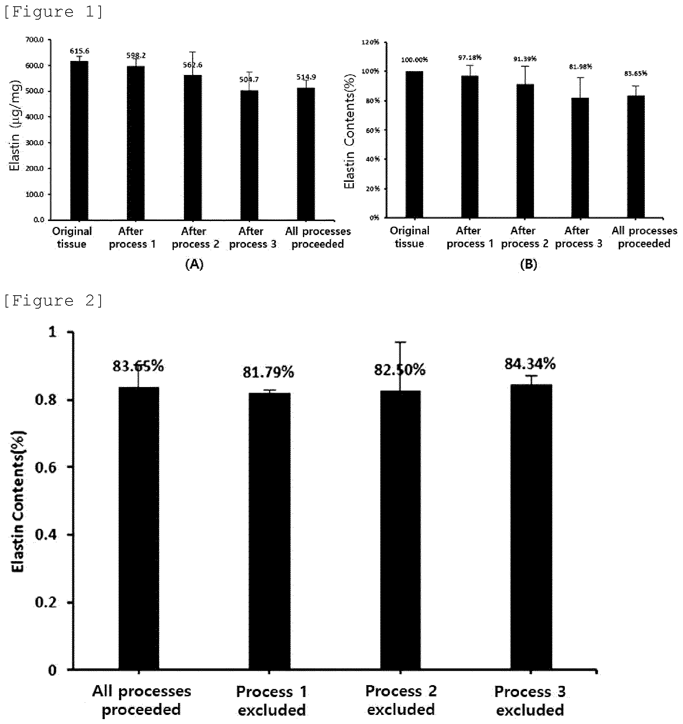

A multi-stage decellularized tissue matrix was prepared using the same method as the Preparative Example, but when the first, second and secondary decellularization steps were performed sequentially, a tissue specimen was collected immediately after each step was performed, and the elastin content per 1 mg of the tissue specimen was measured, and the results are shown in Figure 1(A). In addition, the elastin content in the original tissue was measured in the preparation step, and the elastin content at the time each step was performed relative to the elastin content in the original tissue was expressed as a percentage, and is shown in Figure 1(B).
In Figures 1(A) and 1(B), process 1 refers to the first decellularization step, process 2 refers to the second decellularization step, and process 3 refers to the secondary decellularization step.
For the elastin content, the experiment was performed according to the protocol of the assay kit using the assay kit Fastin Elastin Assay kit F2000 (manufacturer: Biocolor), and then elastin was analyzed at an absorbance of 513 nm using the Multimode plate reader Victor Nivo™ (manufacturer: Perkin Elmer), and the elastin content was measured compared to standard materials.
Referring to the results of Figures 1(A) and 1(B), it could be confirmed that the elastin content was slightly lost as each process progresses, but it could be confirmed that more than about 80% or more of elastin remained at the time the final process is completed.

**[Experimental Example 2]**

Multi-stage decellularized tissue matrices were prepared using the same method as the Preparative Example, but tissue specimens were collected that had progressed to the final process by omitting only one step each of the first, second and secondary decellularization steps, the elastin content per 1 mg of the tissue specimen was measured, the elastin content of the original tissue was measured by the same method in the preparation step, and then the L values were calculated, and the results were listed in Table 1.

In addition, the elastin contents included in the tissue matrices obtained by omitting each step relative to the elastin content in the original tissue in the preparation step were calculated as a percentage, and the results are shown in Figure 2.

The elastin content was measured using the same method as in Experimental Example 1, and in Table 1 and Figure 2, process 1 refers to the first decellularization step, process 2 refers to the second decellularization step, and process 3 refers to the secondary decellularization step.

[Table 1]

| | All processes proceeded | Process 1 excluded | Process 2 excluded | Process 3 excluded |
|---|---|---|---|---|
| $L_0$ (µg/mg) | 615.6 | | | |
| $L_f$ (µg/mg) | 514.9 | 503.5 | 507.8 | 519.1 |
| L (%) | 16.4 | 18.2 | 17.5 | 15.7 |

First, referring to Table 1, it can be confirmed that the L values are 20% or less both when all processes are performed and when one process is omitted, and referring to Figure 2, it can be confirmed that the elastin content does not change significantly even when a specific process is omitted. Therefore, from the results of this experiment, it can be confirmed that even if any one of the first decellularization step, the second decellularization step and the secondary decellularization step is omitted in the process according to one embodiment of the present invention, the elastin content is not significantly affected.

[Experimental Example 3]

The experiment was conducted in the same manner as Experimental Example 1, but the DNA content, rather than the elastin content, was measured after performing each step. The DNA content was measured by performing the experiment according to the protocol of the assay kit using the assay kit Quant-iT™ PicoGreen™ dsDNA Assay kits and dsDNA Reagents (manufacturer: Invitrogen), and then measuring the absorbance at 480-520 nm using the Multimode plate reader Victor Nivo™ (manufacturer: Perkin Elmer).

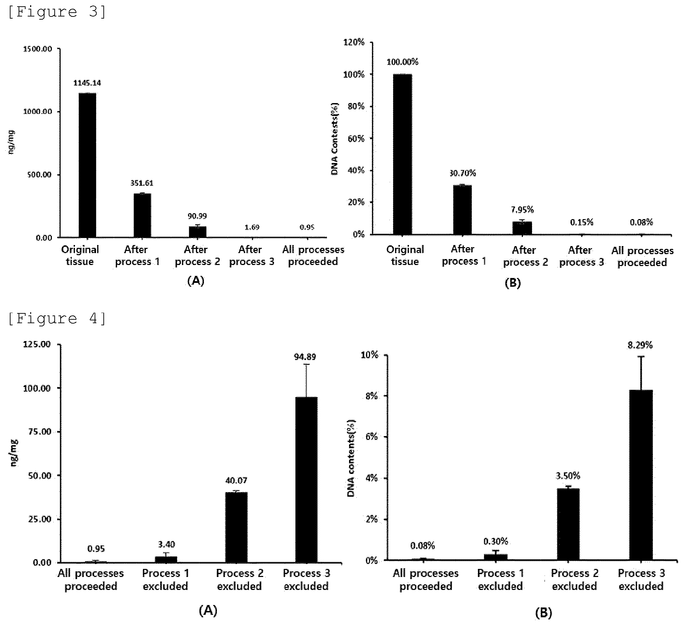

The DNA content per 1 mg of tissue after performing each step is shown in Figure 3(A), and the DNA content of the tissue after performing each step relative to the DNA content of the original tissue is shown as a percentage value in Figure 3(B). In this case, in Figures 3(A) and 3(B), process 1 refers to the first decellularization step, process 2 refers to the second decellularization step, and process 3 refers to the secondary decellularization step.

Referring to Figures 3(A) and 3(B), it can be confirmed that most of the DNA is removed after the first decellularization step, the second decellularization step and the secondary decellularization step are all performed.

[Experimental Example 4]

The experiment was conducted in the same manner as Experimental Example 2, but the DNA contents of the tissue matrices prepared by omitting one step each were measured in the same manner as Experimental Example 3.

The DNA content per 1 mg of the tissue matrix obtained by omitting each step is shown in Figure 4(A), and the DNA content of the tissue obtained by omitting each step relative to the DNA content of the original tissue is shown as a percentage value in Figure 4 (B). In this case, in Figures 4(A) and 4(B), process 1 refers to the first decellularization step, process 2 refers to the second decellularization step, and process 3 refers to the secondary decellularization step.

Referring to Figures 4(A) and 4(B), it can be confirmed that DNA is reliably removed only after the first decellularization step, the second decellularization step and the secondary decellularization step are all performed. In particular, since it can be seen that the DNA removal efficiency is remarkably reduced when the second decellularization step or the secondary decellularization step was omitted, it could be confirmed through this experiment that it is more effective in DNA removal to perform each step sequentially.

[Experimental Example 5]

Multi-stage decellularized tissue matrices were prepared using the same method as in the Preparative Example, but the tissues were treated by changing the concentration of sodium hydroxide contained in the treatment solution in the primary decellularization step to 0.005 M, 0.1 M, 0.2 M and 0.3 M.

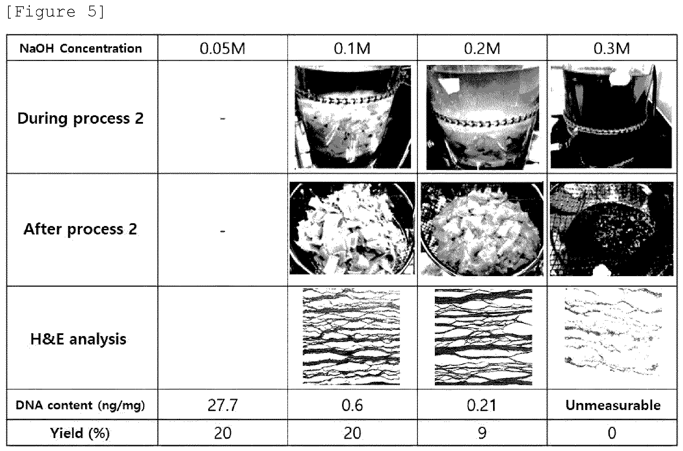

The condition of the tissue during and after the second decellularization step (process 2) treatment during the experiment was photographed and is shown in Figure 5. In addition, the content of DNA included in 1 mg of the finally obtained tissue matrix was measured using the same method as Experimental Example 3, and the tissue matrix yield was calculated by expressing the content of the finally obtained tissue matrix relative to the content of the initially introduced tissue as a percentage value, and the H&E staining photographs were taken and are shown in Figure 5.

Referring to Figure 5, it can be seen that when the concentration of NaOH is 0.05 M, the yield is good, but the DNA content is high, and unremoved cell nuclei were confirmed to exist within the tissue, and thus, the decellularization efficiency is low.

On the other hand, it can be confirmed that when treated with 0.1 M NaOH, the structure of the tissue is well maintained in the second decellularization step, the DNA content after the final treatment is very low at 0.6 ng/mg, and the yield is high at 20.8%.

When treated with 0.2 M NaOH, the DNA content was low, but the final yield was less than half that of when treated with 0.1 M NaOH because some of the tissue collapsed during the primary decellularization step, and when treated with 0.3 M NaOH, tissue could not be obtained because the tissue was almost dissolved.

Therefore, from the results of this experiment, it could be confirmed that in order to obtain a tissue matrix in which the yield was excellent because no collapse or dissolution of the tissue occurred, and DNA within the tissue was sufficiently removed in the primary decellularization step, the concentration of NaOH contained in the treatment solution in the primary decellularization step, particularly in the second decellularization step, was more than 0.05 M and less than 0.2 M.

**[Experimental Example 6]**

Multi-stage decellularized tissue matrices were prepared using the same method as the Preparative Example, but when the concentration of sodium hydroxide contained in the treatment solution in the primary decellularization step was 0.005 M and 0.1 M, the tissues were treated by changing the concentration of DNase to 0.0001 to 0.003 wt%, and then the DNA content ratio and the yield of the tissue matrix of each finally obtained tissue matrix were calculated, and the results are listed in Table 2.

[Table 2]

| NaOH concentration | 0.05 | | | 0.1 | | |
|---|---|---|---|---|---|---|
| DNase concentration (wt%) | 0.0028 | 0.0014 | 0.0007 | 0.0002 | 0.0002 | 0.0001 |
| DNA concentration (ng/mg) | 25.7 | 38.0 | 54.7 | 0.2 | 0.3 | 0.6 |
| Yield (%) | 20.3 | 20.7 | 19.9 | 21.0 | 21.4 | 20.8 |

Referring to the results in Table 2 above, it can be seen that when the concentration of NaOH is constant, the DNA concentration within the final tissue matrix decreases as the concentration of DNase increases. However, it could be confirmed that when the concentration of NaOH was 0.1 M, the DNA concentration in the finally obtained tissue matrix was actually lower and the difference in yield was not significant, even though it was treated with DNase at a concentration several to several tens of times lower than when it was 0.05 M. From the results of this experiment, it can be seen that when the concentration of NaOH was 0.05 M, the DNA removal efficiency was remarkably reduced compared to when it was 0.1 M, and therefore, it is preferable that the concentration of NaOH in the treatment solution used in the primary decellularization step is more than 0.05 M.

The present invention is not limited to the specific embodiments and descriptions described above, and various modifications may be made by those of ordinary skill in the technical field to which the present invention pertains without departing from the gist of the present invention as claimed in the claims, and such modifications fall within the protection scope of the present invention.

[Industrial Availability]

The present invention relates to a multi-stage decellularized tissue matrix having a high elastin content and capable of improving productivity due to high decellularization efficiency, and a method for preparing the same. It may help to rapidly regenerate tissue by promoting re-epithelialization and granulation tissue and new blood vessel formation of damaged tissue due to elastin because the rate of elastin loss is low during the decellularization process. In addition, it may remarkably improve the productivity of the multi-stage decellularized tissue matrix because the decellularization efficiency is excellent and the processing time is short. Therefore, it has industrial applicability.

**Claims**

1.  A multi-stage decellularized mammal-derived tissue matrix, prepared by a method comprising:

    a preparation step for preparing tissue of a non-human mammal;
    a pretreatment step for pretreating the tissue;
    a first inactivation step for inactivating a virus included in the pretreated tissue using alcohol;
    a primary decellularization step for removing cells from the virus-inactivated tissue using an aqueous basic solution;
    a secondary decellularization step for removing cells by enzymatically treating the primary decellularized tissue; and
    a second inactivation step for inactivating a virus included in the decellularized tissue using an acid, and
    wherein the DNA content in the tissue subjected to the second inactivation step is 50 ng/mg or less,
    wherein the reduction rate (L) of the elastin content in the tissue subjected to the pretreatment step to the second inactivation step is 20% or less, and
    wherein the primary decellularization step comprises a first decellularization step performed using a mixture of n-PrOH and NaOH; and a second decellularization step performed using an aqueous sodium hydroxide solution of more than 0.05 M and less than 0.2 M.

2.  The multi-stage decellularized mammal-derived tissue matrix according to claim 1, **characterized in that** the tissue is any one or more of mammal-derived blood vessel, ligament and tendon.

3.  The multi-stage decellularized mammal-derived tissue matrix according to claim 1, **characterized in that** the secondary decellularization step is performed using DNase.

4.  A method for preparing a multi-stage decellularized mammal-derived tissue matrix, comprising:

    a preparation step for preparing tissue of a non-human mammal;
    a pretreatment step for pretreating the tissue;
    a first inactivation step for inactivating a virus included in the pretreated tissue using alcohol;
    a primary decellularization step for removing cells from the virus-inactivated tissue using an aqueous basic solution;
    a secondary decellularization step for removing cells by enzymatically treating the primary decellularized tissue; and
    a second inactivation step for inactivating a virus included in the decellularized tissue using an acid, and
    wherein the DNA content in the tissue subjected to the second inactivation step is 50 ng/mg or less,
    wherein the reduction rate (L) of the elastin content in the tissue subjected to the pretreatment step to the second inactivation step is 20% or less, and
    wherein the primary decellularization step comprises a first decellularization step performed using a mixture of n-PrOH and NaOH; and a second decellularization step performed using an aqueous sodium hydroxide solution of more than 0.05 M and less than 0.2 M.

5.  The method for preparing a multi-stage decellularized mammal-derived tissue matrix according to claim 4, **characterized in that** a neutralization step using an acidic solution and a washing step using PBS are further performed between the primary and secondary decellularization steps.

6.  The method for preparing a multi-stage decellularized mammal-derived tissue matrix according to claim 4, **characterized in that** a decolorization step and/or a defatization step are further performed between the secondary decellularization step and the second inactivation step.

7.  The method for preparing a multi-stage decellularized mammal-derived tissue matrix according to claim 4, **characterized in that** the second inactivation step is performed using a mixture of an organic acid and an alcohol.

8.  The method for preparing a multi-stage decellularized mammal-derived tissue matrix according to claim 4, **characterized in that** washing, packaging and sterilization steps are performed after the second inactivation step.

[Figure 1]

(A)

(B)

[Figure 2]

[Figure 3]

(A)

(B)

[Figure 4]

(A)

(B)

[Figure 5]

| NaOH Concentration | 0.05M | 0.1M | 0.2M | 0.3M |
|---|---|---|---|---|
| During process 2 | - | | | |
| After process 2 | - | | | |
| H&E analysis | | | | |
| DNA content (ng/mg) | 27.7 | 0.6 | 0.21 | Unmeasurable |
| Yield (%) | 20 | 20 | 9 | 0 |

| INTERNATIONAL SEARCH REPORT | International application No. |
|---|---|
| | **PCT/KR2023/002941** |

**A. CLASSIFICATION OF SUBJECT MATTER**

**A61L 27/36**(2006.01)i; **A61L 27/40**(2006.01)i

According to International Patent Classification (IPC) or to both national classification and IPC

**B. FIELDS SEARCHED**

Minimum documentation searched (classification system followed by classification symbols)

A61L 27/36(2006.01); A61F 2/00(2006.01); A61F 2/02(2006.01); A61F 2/08(2006.01); A61L 27/00(2006.01); A61L 27/38(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 탈세포(decellularization), 포유류(mammal), 불활성화(inactivation), 염기(base), 산(acid), 효소(enzyme), 엘라스틴(elastin), DNA

**C. DOCUMENTS CONSIDERED TO BE RELEVANT**

| Category* | Citation of document, with indication, where appropriate, of the relevant passages | Relevant to claim No. |
|---|---|---|
| A | KR 10-2009-0088054 A (BIOLAND LTD.) 19 August 2009 (2009-08-19) See paragraphs [0014]-[0018], [0023]-[0030], [0043] and [0070]; and table 4. | 1-8 |
| A | KR 10-2014-0007192 A (OSCOTEC INC.) 17 January 2014 (2014-01-17) See entire document. | 1-8 |
| A | LI, T. et al. A saponification method for chlorophyll removal from microalgae biomass as oil feedstock. Marine drugs. 2016, vol. 14, no. 9, article no. 162, pp. 1-19. See entire document. | 1-8 |
| A | KR 10-0587868 B1 (ORGANOGENESIS, INC.) 13 June 2006 (2006-06-13) See entire document. | 1-8 |
| A | JP 2012-166039 A (APERION BIOLOGICS INC.) 06 September 2012 (2012-09-06) See entire document. | 1-8 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 June 2023** | **15 June 2023** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/002941**

| Patent document cited in search report | | | Publication date (day/month/year) | Patent family member(s) | | | Publication date (day/month/year) |
|---|---|---|---|---|---|---|---|
| KR | 10-2009-0088054 | A | 19 August 2009 | CN | 101507837 | A | 19 August 2009 |
| | | | | KR | 10-0947553 | B1 | 12 March 2010 |
| | | | | US | 2009-0208551 | A1 | 20 August 2009 |
| KR | 10-2014-0007192 | A | 17 January 2014 | KR | 10-1455837 | B1 | 03 November 2014 |
| | | | | WO | 2014-010859 | A1 | 16 January 2014 |
| KR | 10-0587868 | B1 | 13 June 2006 | AT | 281128 | T | 15 November 2004 |
| | | | | AU | 7294798 | A | 27 November 1998 |
| | | | | AU | 736312 | B2 | 26 July 2001 |
| | | | | BR | 9809216 | A | 27 June 2000 |
| | | | | BR | 9809216 | B1 | 09 March 2010 |
| | | | | CA | 2288823 | A1 | 12 November 1998 |
| | | | | CA | 2288823 | C | 31 July 2007 |
| | | | | CN | 1267201 | A | 20 September 2000 |
| | | | | DE | 69827402 | T2 | 03 November 2005 |
| | | | | DK | 1018979 | T3 | 14 February 2005 |
| | | | | EP | 1018979 | A1 | 19 July 2000 |
| | | | | EP | 1018979 | A4 | 09 August 2000 |
| | | | | EP | 1018979 | B1 | 03 November 2004 |
| | | | | EP | 1452150 | A1 | 01 September 2004 |
| | | | | EP | 1452150 | B1 | 09 January 2013 |
| | | | | ES | 2231984 | T3 | 16 May 2005 |
| | | | | ES | 2404033 | T3 | 23 May 2013 |
| | | | | JP | 4558107 | B2 | 06 October 2010 |
| | | | | KR | 10-2001-0012287 | A | 15 February 2001 |
| | | | | NO | 995372 | L | 03 November 1999 |
| | | | | PT | 1018979 | E | 28 February 2005 |
| | | | | US | 2004-0005703 | A1 | 08 January 2004 |
| | | | | US | 2006-0024380 | A1 | 02 February 2006 |
| | | | | US | 5993844 | A | 30 November 1999 |
| | | | | US | 6599690 | B1 | 29 July 2003 |
| | | | | US | 6893653 | B2 | 17 May 2005 |
| | | | | WO | 98-49969 | A1 | 12 November 1998 |
| JP | 2012-166039 | A | 06 September 2012 | AU | 2985700 | A | 29 August 2000 |
| | | | | AU | 760424 | B2 | 15 May 2003 |
| | | | | CA | 2361579 | A1 | 17 August 2000 |
| | | | | EP | 1158930 | A1 | 05 December 2001 |
| | | | | JP | 2002-536109 | A | 29 October 2002 |
| | | | | JP | 5015376 | B2 | 29 August 2012 |
| | | | | JP | 5654516 | B2 | 14 January 2015 |
| | | | | US | 2001-0039459 | A1 | 08 November 2001 |
| | | | | US | 6267786 | B1 | 31 July 2001 |
| | | | | US | 6455309 | B2 | 24 September 2002 |
| | | | | WO | 00-47131 | A1 | 17 August 2000 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101410533